Europäisches Patentamt
European Patent Office
Office européen des brevets

(11) Publication number: 0 385 952 A2

# EUROPEAN PATENT APPLICATION

(21) Application number: 90810157.9

(51) Int. Cl.5: A61K 31/135, C07C 215/54

(22) Date of filing: 01.03.90

(30) Priority: 03.03.89 DE 3906720

(43) Date of publication of application: 05.09.90 Bulletin 90/36

(84) Designated Contracting States: AT BE CH DE DK ES FR GB GR IT LI LU NL SE

(71) Applicant: SANDOZ AG
Lichtstrasse 35
CH-4002 Basel(CH)
(84) BE CH DK ES FR GB GR IT LI LU NL SE

Applicant: SANDOZ-PATENT-GMBH
Humboldtstrasse 3
D-7850 Lörrach(DE)
(84) DE

Applicant: SANDOZ-ERFINDUNGEN Verwaltungsgesellschaft m.b.H.
Brunner Strasse 59
A-1235 Wien(AT)
(84) AT

(72) Inventor: Cooper, Philip Henry
Myrtenweg 31
CH-3018 Berne(CH)
Inventor: Mieth, Hubert
Ferdinandsgasse 16
A-2351 Wiener Neudorf(AT)
Inventor: Richter, Friedrich
Mattenweg 4
CH-3322 Schönbühl-Urtenen(CH)
Inventor: Schuster, Ingeborg
H. Maierstrasse 60/5
A-1180 Vienna(AT)
Inventor: Stütz, Anton
Lindauergasse 35
A-1238 Vienna(AT)

(54) **Use of a naphthalenmethanamino derivative as antiinflammatory agent and pharmaceutical compositions containing it.**

(57) The compound of formula I

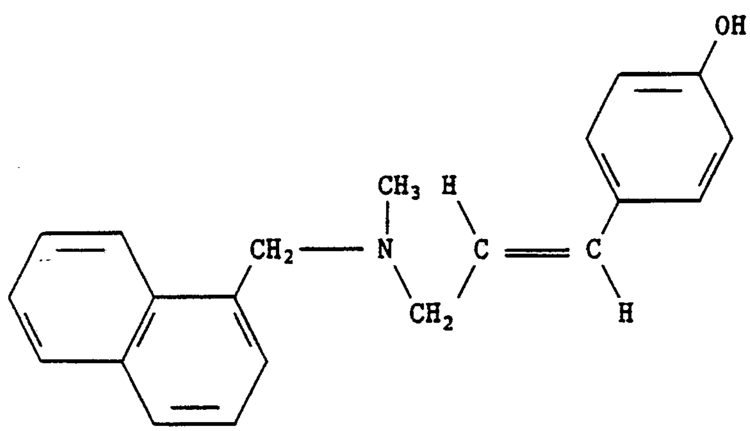

I

in free base form or in naphthalene-1,5-disulfonate acid addition salt form is known. It has now been found to possess anti-inflammatory activity. It is thus indicated for anti-inflammatory use, especially topically. Further, water-soluble acid addition salt forms have been found which are indicated for use in the

EP 0 385 952 A2

preparation of e.g. pharmaceutical compositions wherein the compound is present in dissolved state in an aqueous phase.

# USE OF A NAPHTHALENMETHANAMINO DERIVATIVE AS ANTIINFLAMMATORY AGENT AND PHARMACEUTICAL COMPOSITIONS CONTAINING IT

## A. FIELD

The invention relates to the field of naphthalenmethanamino derivatives. It concerns the use of the compound of formula I

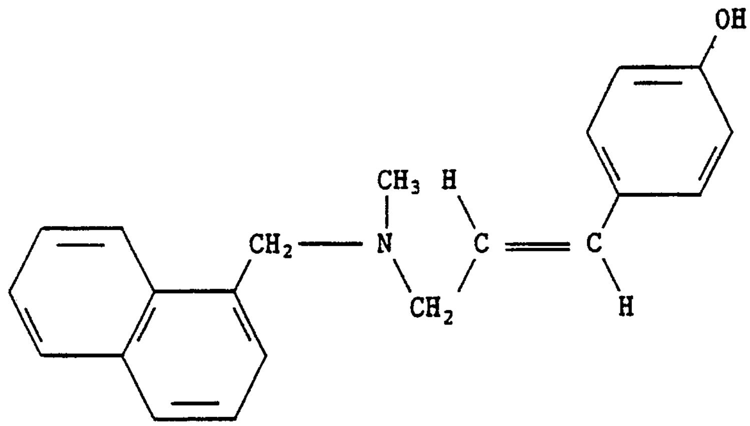

I

i.e. of (E)-N-methyl-N-[3-(4-hydroxyphenyl)-2-propenyl]-1-naphthalene-methanamine or, alternatively, (E)-N-methyl-N-(1-napthylmethyl)-3-(4-hydroxyphenyl)-prop-2-en-1-amine, in free base form or in acid addition salt form, hereinafter designated briefly as "the compound of the invention", in the treatment of inflammatory conditions, in particular in the topical treatment of inflammatory skin diseases; water-soluble acid addition salt forms of the compound of formula I; and pharmaceutical compositions wherein the compound of the invention is e.g. present in dissolved state in an aqueous phase.

## B. STATE OF THE ART

One of the most important objectives in dermatological drug research is the identification and development of active principles with corticoid-like beneficial effects, but devoid of the undesirable cutaneous and systemic liabilities of corticosteroids. Such drugs are named non-steroidal anti-inflammatory drugs (NSAID). The cyclooxygenase inhibitors as classical NSAID's have failed in dermatology. The clinical potential of lipoxygenase inhibitors, on the other hand, is still unproven. While bufexamac, i.e. 2-[p-(n-butyloxy)phenyl]-acetohydroxamic acid is the sole topical NSAID to date in clinical use, its dermatological efficacy has been questioned [R. Trancik & N.J. Lowe, Non-Steroidal Anti-Inflammatory Drugs, Eds. Lowe N.J., Hensby, C.N., Karger, Basel (1989) p. 136-147].

There is thus a need for further compounds with distinctive anti-inflammatory properties, structurally and mechanistically unrelated to classical anti-inflammatories.

The compound of formula I in free base and naphthalin-1,5-disulfonate acid addition salt form, its preparation and its use as an antifungal agent are known from e.g. USP 4'282'251. It is specifically disclosed as Example 16 therein. It can be prepared e.g. according to process d), analogously to Example 4 therein, by splitting off water from N-methyl-N-(1-naphthylmethyl)-3-(4-hydroxyphenyl)-propan-3-ol-1-amine. The starting material can be prepared by reaction of N-methyl-1-naphthalene-methanamine with 4-hydroxyacetophenone and reduction of the resultant N-methyl-N-(1-naphthylmethyl)-3-(4-hydroxyphenyl)-propan-3-on-1-amine.

## C. SUMMARY OF THE INVENTION

It has now been found that in addition to its known antimycotic activity the compound of the invention possesses further interesting pharmalogical properties and is therefore indicated for further uses as a

pharmaceutical. The compound of the invention, surprisingly, meets all the requirements for a NSAID and possesses a unique profile of activity.

Further, water-soluble acid addition salt forms of the compound of the invention have been found from which novel galenical forms, e.g. pharmaceutical compositions wherein the compound is present in dissolved state in an aqueous phase can be obtained having beneficial properties.

The compound of formula I may thus be in free base form or in acid addition salt form, e.g. the lactate, the ascorbate or the naphthalen-1,5-disulfonate form. Preferred are the free base form (M.P. 135-137° C), the lactate and the ascorbate.

## D. DETAILED DESCRIPTION

### ABBREVIATIONS:

Con A: Concanavalin A
DAE: Dimethylacetamide, acetone and ethanol (2:4:4)
DMSO-$d_6$: Deuterated dimethylsulfoxide
FCS: 10 % fetal calf serum
IL: Interleukin
KGM: Keratinocyte growth medium
LPS: Lipopolysaccharide
$LTC_4$: Leukotriene $C_4$
NAP-1: Neutrophil activating protein-1
NSAID: Non-steroidal anti-inflammatory drug
Pen/strep: Penicillin/streptomycin
$PGE_2$: Prostaglandin-$E_2$
$\alpha$TNF: Tumor necrosis factor-$\alpha$
TPA: 12-O-Tetradecanoylphorbol-13-acetate
$TXB_2$: Thromboxane $B_2$

### EXPLANATION OF THE FIGURES:

Figure 1: NMR spectrum in DMSO-$d_6$ of the compound of the invention in lactate acid addition salt form after salt formation in deuterated water (see Example 10).

Figure 2: NMR spectrum in DMSO-$d_6$ of the compound of the invention in lactate acid addition salt form after salt formation with an equimolar amount of lactic acid (see Example 11).

Figure 3: NMR spectrum in DMSO-$d_6$ of the compound of the invention in free base form (see Example 11).

Figure 4: NMR spectrum in DMSO-$d_6$ of the compound of the invention in lactate acid addition salt form after salt formation with a 1.5 molar excess of lactic acid (see Example 12).

Figure 5: Photograph of the compound of the invention in free base form, before micronisation (1cm = 55 $\mu$m)

Figure 6: Photograph of the material of Figure 5 after recrystallization from ethanol/water 1:1 (1 cm = 55 $\mu$m).

Figure 7: Photograph of a cream prepared according to Example 5 but with b) omitted (1 cm = 55 $\mu$m).

Figure 8: Photograph of the cream prepared according to Example 5 (1 cm = 55 $\mu$m).

### PHARMACOLOGICAL ASPECTS

The compound of the invention:

**1.** in vitro:

- inhibits macrophage activation, as can be determined with the release of plasminogen activator, hydrogen peroxide and prostaglandin-related mediators, at concentrations of from about 1 $\mu$m to about 8 $\mu$m;
- inhibits lps-induced transcription of nap-1/il-8, $\alpha$tnf and il-i$\beta$ in the macrophage-like mono-mac 6 cell line, at a concentration of about 10 $\mu$m;

- inhibits tpa-stimulated $pge_2$ synthesis in post-confluent cultures of human keratinocytes, at concentrations of from about 1 µm to about 3 µm;
- drastically reduces nap1/il-8 mrna accumulation and protein release induced by tgf-α, αtnf, il-1α or tpa in hacat keratinocytes, at concentrations of from about 10 µm to about 20 µm;

2. in vivo:
- shows excellent anti-inflammatory activity in various test models, particularly upon topical application, e.g. in irritant contact dermatitis models, in the UV-erythema model and in the contact allergic dermatitis model, at concentrations of from about 1 % to about 5 %.

In contrast thereto, the compound of the invention does not inhibit, or inhibits only to a small extent, representative functions of
- neutrophils: the $IC_{50}$ for FMLP-stimulated release of specific granule, azurophil granule and lysosomal enzymes in the neutrophil exocytosis assay, and for zymosan-stimulated superoxide release from human neutrophils is more than 100 µM; for 5-lipoxygenase, 5,12-lipoxygenase and 12 + 15-lipoxygenase it is 44 µM, 45 µM and, respectively, 100 µM;
- platelets: the $IC_{50}$ for thrombin-stimulated release of serotonin and N-glucosaminidase from bovine platelets is above 100 µM and for thromboxane $B_2$ production it is 100 µM; and
- mast cells: the $IC_{50}$ for polymyxin-stimulated release of histamine and β-glucosaminidase is above 100 µM.

Cyclooxygenase (prostaglandin synthetase) is not inhibited at 100 µM and the compound does thus not belong to the family of cyclooxygenase inhibitors. The effects observed are not caused by cytotoxicity since release of lactate dehydrogenase is below 10 %.

The above test methods and results are hereinafter set out in more detail (in all pharmacological assays the compound of the invention is used in free base form; in all in vitro assays the compound is dissolved in dimethylsulfoxide at a concentration of $10^{-2}$ M unless stated otherwise hereinafter):

## 1. IN VITRO ASSAYS

### 1.1. Inhibition of macrophage activation

#### 1.1.1. Inhibition of Con A-stimulated plasminogen activator release

Macrophages 10 to 14 days old isolated from the bone marrow of mice are incubated in monolayers with the test compound in the presence of Concanavalin A (Con A) (100 µg/ml) as soluble stimulant. After 24 hours the cell-free medium is collected and macrophage activation is investigated biochemically as the extent of plasminogen activator release, assayed photometrically as the plasminogen-dependent amidolysis of D-Val-Phe-Lys-4-nitroanilide according to the principles described in J.C. Drapier et al., Biochemie 61 - (1979) 463-471. Cell viability is assayed by measurement of lactate dehydrogenase release according to the method of H.U. Bergmeyer and E. Bernt, Methoden der Enzymatischen Analyse, 3. Auflage, Verlag Chemie (1974) 612-615, adapted as a kinetic assay using microtiter plates. All cultures are checked microscopically for qualitative morphological modification.

The compound of the invention has an $IC_{50}$ of 1.8 µM in this assay.

#### 1.1.2. Zymosan-stimulated release of hydrogen peroxide

The production of hydrogen peroxide is used as a measure for the respiratory burst of phagocytic cells. Macrophages 10 to 14 days old, isolated from the bone marrow of mice are incubated for 10 to 30 minutes in the presence of the test compound. Zymosan (0.5 ml) is then added as phagocytic stimulant and the mixture is incubated for a further 2 hours at 37° C in the presence of homovallinic acid (0.5 ml, 400 µM) and horse radish peroxidase (4 U/ml). The reaction is stopped by the addition of 0.25 ml 0.1 M glycine-NaOH buffer, pH 12, containing 25 mM EDTA, the samples centrifuged at 2200 g for 10 minutes, and the homovallinic acid oxidation product, as a measure of the amount of $H_2O_2$ produced, measured fluorimetrically.

In this assay the compound of the invention has an $IC_{50}$ of 2.1 µM.

**1.1.3.** Zymosan-induced release of thromboxane $B_2$ ($TXB_2$), prostaglandin $E_2$ ($PGE_2$) and leukotriene $C_4$ ($LTC_4$)

The test compound is added in various concentrations to monolayers of $OF_1$-mouse resident peritoneal macrophages isolated as described in J. Schnyder and M. Baggiolini, J. Exp. Med. 148 (1978) 435-450. 0.5 ml of the suspension containing 1.2 x $10^6$ cells/ml DMEM (Dulbecco's modified Eagles medium, non-essential amino acids, 2 mM L-glutamine, 100 U/ml penicillin/streptomycin) containing 2 % heat-inactivated fetal calf serum and 20 U heparin/ml is added to each well of 24-well plates (diameter 1.6 cm) and allowed to adhere at 37° C for 2 hours in 5 % $CO_2$/air. The monolayers are then washed with PBS and 0.5 ml DMEM containing 1 % heat-inactivated fetal calf serum added. Various concentrations of the test substance in the same medium (containing a maximum final concentration of 1 % DMSO) are added, the final volume being 0.98 ml, and after 10 minutes at 37° C, the cells are stimulated by addition of 20 µl of a 1-20 v/v dilution of zymosan particles (Sigma Chemie, Deisenhofen, FRG) in 50 mg/ml PBS. After 2 hours at 37° C, the media are removed, centrifuged at 1300 g for 5 minutes and decanted. The cells are lysed with 1ml 0.01 % digitonin. The supernatants are then analysed by radioimmunoassay procedures for thromboxane $B_2$, prostaglandin $E_2$ and leukotriene $C_4$. Lactate dehydrogenase activities in the medium and cell lysates are also measured as an indication of cell toxicity.

In this assay the compound of the invention has an $IC_{50}$ of 6.0 µM for $TXB_2$, of 1.5 µM for $PGE_2$ and of 8.0 µM for $LTC_4$.

**1.1.4.** TPA-induced prostaglandin $E_2$ ($PGE_2$) release

Peritoneal exudate cells of NMRI mice (strain NMRI) pretreated with 1.5 ml thioglycollate ip 3 days previously are harvested by peritoneal lavage, washed in phosphate-buffered saline (PBS without $Ca^{++}$ and $Mg^{++}$) and resuspended in DMEM medium supplemented with 10 % fetal calf serum (FCS). 1x$10^6$ cells in 1 ml are placed into wells of Costar-24 tissue culture plates and cells are allowed to adhere for 24 hours at 37° C and 5 % $CO_2$. Cells are then washed twice with PBS and the remaining, more than 95 % pure macrophage population is stimulated with 12-O-tetradecanoylphorbol-13-acetate (TPA) (20 ng/ml, 1h) in DMEM medium without FCS. Conditioned media are centrifuged and $PGE_2$ levels are determined using a $^{125}I$ radioimmunoassay. Samples are assayed in duplicate. Inhibition of $PGE_2$ release by test compounds is calculated as percent inhibition compared to the controls.

The $IC_{50}$ for the inhibition found in this assay with the compound of the invention is 1 to 3 µM.

**1.2. LPS-induced NAP1/IL-8, αTNF- and IL-1β-mRNA accumulation in the human cell line Mono-Mac-6**

Human Mono-Mac-6 cells, which have many of the phenotypic and functional characteristics of mature peripheral blood monocytes [H. Ziegler et al, Int. J. Cancer 41 (1988) 456-461] are grown up to $10^6$ cells/ml im RPMI 1640 medium supplemented with 10 % FCS, 1 % pen/strep and 0.25 % amphotericin. In each of 6 activation experiments $10^7$ cells are incubated for 4 hours at 37° C and 5 % $CO_2$. LPS is used in a concentration of 1 µg/ml and the test substance in concentrations of $10^{-5}$ M to $10^{-7}$ M.

RNA blotting analysis:

After induction times the cells are pelleted [5 min, 1200 rpm, room temperature]. Total cellular RNA is extracted from the pelleted cells using the guanidinium isothiocyanate/cesium chloride method of J.M. Chirgwin et al., Biochemistry 18 (1979) 5294-5299. 10 µg samples of the extracted RNA are size-fractionated by 1.2 % formaldehyde agarose gel electrophoresis [T. Maniatis et al., Molecular Cloning: A Laboratory Manual, Cold Spring Harbour, New York (1982) p. 187-206], transferred to synthetic membranes (Hybond N, Amersham) with 20xSSC (1xSSC is 150 mM NaCl, 15 mM sodium citrate, pH 7.0) overnight. The filters are baked for 2 hours at 65° C and prehybridized for 4 hours at 65° C in 5xSSC, 10x Denhardt's solution (1x Denhardt's solution is 0.02 % bovine serum albumin, 0.02 % polyvinyl pyrrolidone, 0.02 % ficoll), 10 % dextrane sulphate, 20 mM sodium phosphate pH 7.0, 7 % SDS, 100 µg/ml sonicated salmon sperm DNA, and 100 µg/ml polyA. $^{32}P$-labelled synthetic NAP1/IL8, αTNF and IL-1β oligonucleotide probes are used as the hybridization probes. Hybridization is performed at 65° C in the pre-hybridization buffer

containing the labelled probe for 16 hours. The blots are washed once in 5 % SDS, 3xSSC, 10x Denhardt's solution, 20 mM sodium phosphate pH 7.0 for 30 minutes at 65 °C, and once in 1xSSC, 1 % SDS at 65 °C for 30 minutes. Filters are exposed at -70 °C to KODAK XAR-5 films using intensifying screens. After washing for 30 minutes in 0.1 % SDS at 65 °C, blots are reused for a second hybridization with a $\beta$-actin specific oligonucleotide probe to quantitate the mRNA amounts.

In this assay the compound of the invention decreases LPS-induced NAP1/IL-8-, $\alpha$TNF- and IL-1$\beta$-mRNA accumulation to 20 %, 10 % and 50 %, respectively, at a concentration of 10.0 $\mu$M.

### 1.3. Inhibition of TPA-stimulated prostaglandin E2 ($PGE_2$) synthesis of human keratinocytes

Cultures of human keratinocytes are obtained by trypsinisation of neonatal human foreskins. The keratinocyte cultures are grown in a supplemented keratinocyte growth medium (KGM; Clonetics) using culture flasks. Passages 3-5 of 80-90 % confluent keratinocytes are resuspended in KGM at a concentration of $1x10^5$ cells/ml and 0.1 ml are plated into 96 well-microtiter plates in the presence of test compound. For the stimulation of $PGE_2$ release human keratinocytes or the HACAT human skin keratinocyte cell line [P. Boukamp et al., J. Cell Biol. 106 (1988) 761-771] are grown to confluence in KGM medium and stimulated with TPA (100 ng/ml) for 24 hours. Conditioned media are centrifuged and concentrations of $PGE_2$ are determined using a $^{125}I$ radioimmunoassay (NEN, Boston, Mass.). Each sample is assayed in duplicate.

In this assay the compound of the invention inhibits $PGE_2$ synthesis at an $IC_{50}$ of from 1 $\mu$M to 3 $\mu$M.

### 1.4. Modulation of keratinocyte gene expression and NAP1/IL-8 protein release

The HACAT cell line is grown in 200 ml culture flasks in KGM medium (Clonetics) at 0.06 mM Ca until 50 % confluency. Thereafter cells are starved in KBM medium (Clonetics) for 2 days. The resulting preconfluent cells are then stimulated with $\alpha$TNF (Genzyme, 500 or 1000 U/ml) or IL-1$\alpha$ (Boehringer, 200 U/ml) obtained from stock solutions and further diluted in KBM medium for the indicated times in the presence of test substance. At the end of the incubation period total cellular RNA is extracted as described above under 1.1.5. 20 $\mu$g RNA is run on 1 % formaldehyde agarose gels and transferred onto hybond N filters (Amersham) in a northern capillary transfer using 20xSSC. The filters are then baked for 2 hours and prehybridized for 4 hours. Hybridization (16 hours) and washing are performed at 65 °C using oligonucleotide probes for NAP1/IL-8. For control, blots are rehybridised with an actin probe (Oncor). Concentrations of NAP1/IL-8 in the supernatants of the keratinocyte cultures are determined using a solid phase double ligand ELISA.

In this assay the compound of the invention decreases $\alpha$TNF-induced NAP1/IL-8 mRNA accumulation to 60 % at a concentration of 20.0 $\mu$M and inhibits $\alpha$TNF- and IL-1$\alpha$-stimulated release of NAP1/IL-8 by 90 % and, respectively, 40 % at a concentration of 10.0 $\mu$M.

## 2. IN VIVO ASSAYS

### 2.1. Irritant contact dermatitis models

#### 2.1.1. Inhibition of arachidonic acid- or cantharidin-induced pinnal swelling in mice

Skin irritation with arachidonic acid or cantharidin is often used to experimentally induce inflammation in order to evaluate the anti-inflammatory activity of a compound [see e.g. Inflammation 8 (1984) 134-135; 10 (1986) 205-214]. NMRI mice (8 animals per group) receive 10 $\mu$l of a 10 % solution of arachidonic acid [J.M. Young et al., J. Invest. Derm. 82 (1986) 367-371] in acetone or, respectively, 0.08 % cantharidin [K.E. Swingle et al., Arch. Int. Pharmacodyn. Ther. 254 (1981) 168-176] in acetone applied to the medial and lateral surface of the right external ear. Treatment is performed either prophylactically 30 minutes before irritation (arachidonic acid) or, respectively, either simultaneously with the application of irritant solution or therapeutically 20 minutes after irritation (cantharidin), by a single epicutaneous application of the test compound dissolved in a mixture of N,N-dimethylacetamide, acetone and alcohol (2:2:4). The evaluation of the test group is effected by comparison with a control group of animals having received on the right ear

lobe only the carrier substance or the irritant solution. The animals are killed 1.5 hours (arachidonic acid) or, respectively, 17 hours (cantharidin) after application of the irritant and efficacy in assessed by determination of the differences in auricular weight. Differences in weights (D) of right (involved) and left (uninvolved) pinnae of animals treated with test compound and arachidonic acid or cantharidin (T-group) are compared with differences in mice treated with arachidonic acid only or cantharidin only (C-group). Efficacy (%) is calculated according to the equation:

$$\% = \frac{100 \text{ x } D_C \text{minus} D_T}{D_C}$$

2.1.2. Inhibition of croton oil- or TPA-induced pinnal swelling in mice

This model is similar to the above (see 2.1.1.). Skin inflammation is induced with 15 µl of 0.23 % croton oil in a mixture of dimethylacetamide, acetone and ethanol (2:4:4), or with 10 µl of 0.005 % TPA [12-O-tetradecanoylphorbol-13-acetate; Sigma] prepared in acetone with 0.5 % dimethylsulfoxide. Topical treatment is performed either simultaneously with the irritation (croton oil) or 20 minutes after the application (TPA). The test compound is prepared in DAE. Efficacy is assessed 6 hours after onset of irritation by determination of the auricular weight as described above.

2.2. UV-erythema model

Female guinea pigs weighing 250-300 g are used. On the day before experiment they are shorn on both flanks followed by a final dry shaving without skin irritation two hours before UV exposure. Two circular areas (10 mm diameter) on each flank are exposed 10 seconds to ultraviolet rays from 250-500 mm at an intensity of 16 $mW/cm^2$ (UVA) and 500 $mW/cm^2$ (UVB). Drugs are administered topically immediately after exposure to UV light in a volume of 50 µl of 25 % ethanol and 75 %polyethylenglycol-400 to the irradiated skin areas on the right flank. Two areas on the left flank serve as vehicle control. Erythema is assessed visually 3.5 and 5.5 hours after irradiation and results expressed in scores. Changes in erythema intensity after drug administration are expressed as percentages of the scored erythema intensity in vehicle-treated areas.

2.3. Allergic contact dermatitis model

The inhibition of allergic contact dermatitis to oxazolone in mice is determined. Female, 5 weeks old NMRI mice (8 per group) are sensitized with 10 µl of 2 % oxazolone [F.M. Dietrich and R. Hess, Int. Arch. Allergy 38 (1970) 246-259] applied to the shaved ventral abdomen. Challenge reaction is provoked after 7 days by application of 10 µl of 2 % oxazolone to the pinnae. Topical application of the test compound (dissolved in DAE) is performed twice, 20 minutes and 2 hours after challenge. 24 hours after skin testing treatment-related inhibition of contact dermatitis is assessed by pinnal weight as described above (see 2.1.1.).

The following results are obtained in the above in vivo assays:

| Assay | Irritant or allergen | Mode of treatment | Test concentration (%) | Efficacy (%) Compound of the invention | Efficacy (%) Bufexamac |
|---|---|---|---|---|---|
| Contact dermatitis (2.1.1.) | arachidonic acid | prophylactic | 1.2<br>3.6 | 33**<br>48*** | 35*<br>22* |
| Contact dermatitis (2.1.1.) | cantharidin | simultaneous | 1.2<br>3.6 | 26<br>52** | 36<br>29 |
| | | therapeutic | 1.2<br>3.6 | 25<br>50** | 22<br>14 |
| Contact dermatitis (2.1.2.) | croton oil | simultaneous | 1.2<br>3.6 | 67**<br>81** | 11<br>36** |
| Contact dermatitis (2.1.2.) | TPA | therapeutic | 1.2<br>3.6 | 51<br>60*** | 1<br>2 |
| UV-erythema (2.2.) | | therapeutic | 5.0 | 27 | 8 |
| Contact dermatitis (2.3.) | oxazolone | therapeutic | 3.6 | 42** | ø |

* $P < 0.05$
** $P < 0.01$
*** $P < 0.001$

ø no activity

| Assay | Irritant or allergen | Mode of treatment | Test concentration (%) | Efficacy (%) | |
|---|---|---|---|---|---|
| | | | | Compound of the invention | Bufexamac |
| Contact dermatitis (2.1.1.) | arachidonic acid | prophylactic | 1.2 | $33^{**}$ | $35^{*}$ |
| | | | 3.6 | $48^{***}$ | $22^{*}$ |
| Contact dermatitis (2.1.1.) | cantharidin | simultaneous | 1.2 | 26 | 36 |
| | | | 3.6 | $52^{**}$ | 29 |
| | | therapeutic | 1.2 | 25 | 22 |
| | | | 3.6 | $50^{**}$ | 14 |
| Contact dermatitis (2.1.2.) | croton oil | simultaneous | 1.2 | $67^{**}$ | 11 |
| | | | 3.6 | $81^{**}$ | $36^{**}$ |
| Contact dermatitis (2.1.2.) | TPA | therapeutic | 1.2 | 51 | 1 |
| | | | 3.6 | $60^{***}$ | 2 |
| UV-erythema (2.2.) | | therapeutic | 5.0 | 27 | 8 |
| Contact dermatitis (2.3.) | oxazolone | therapeutic | 3.6 | $42^{**}$ | ∅ |

$^{*}$ $P < 0.05$
$^{**}$ $P < 0.01$
$^{***}$ $P < 0.001$
∅ no activity

As appears from the above, while the in vitro data obtained indicate a unique activity profile of the compound of the invention, in vivo the compound of the invention is not only highly active but also clearly superior to bufexamac, the sole currently available non-steroidal compound for topical treatment of inflammatory skin diseases.

**CLINICAL ASPECTS**

A clinical trial of the compound of the invention is effected as follows:

The trial is conducted employing a group of volunteers (mixed male and female of average body weight) identified as exhibiting dermal inflammation. Subjects selected are primarily selected from long-term sufferers and non-responders to conventional therapy. Each subject receives a composition in accordance with the invention, e.g. a 1 % cream. Compositions are applied topically to a site of inflammatory lesion in an amount of from about 0.005 $g/cm^2$ to about 0.05 $g/cm^2$. Application is effected 1, 2 or 3 times daily depending on the extent of lesion. Treatment is continued for each subject for a total period of at least 2 weeks. Alternative treatment is withdrawn prior to and during topical treatment with the compound of the invention. Each subject undergoes full dermatological examination prior to commencement of treatment to determine extent, location and severity of inflammatory lesions. Each subject is also questioned to determine subjective experience of the disease. Examination is repeated at weekly intervals and again at the conclusion of treatment and all changes in condition are noted. At the conclusion of treatment each subject is again questioned to determine subjective experience of the disease. All changes in the subjects' condition, especially extent, intensity of lesion as well as any side effects, are noted. Results obtained on administration of composition in accordance with the invention are compared with those obtained for a control group receiving a placebo composition not comprising the compound of the invention. Results obtained show marked reduction of inflammation in subjects receiving compositions in accordance with the invention administered as described as compared with control groups receiving placebo. Compositions in accordance with the invention tested are found to be well tolerated.

The compound of the invention is thus indicated for use as an anti-inflammatory agent. It is particularly indicated for use as an anti-inflammatory dermatic, especially for the treatment of inflammatory conditions of mycotic or, preferably, non-mycotic etiology, especially of inflammatory skin diseases such as various forms of eczema, irritant dermatitis, allergic contact dermatitis and UV erythema. Preferred is the treatment of eczema.

Particularly preferred is topical treatment.

The compound of the invention may be used in free base form or in pharmaceutically acceptable acid addition salt form.

For the above indications the appropriate dosage will, of course, vary depending upon, for example, the host, the mode of administration and the nature and severity of the condition being treated. However, in general, satisfactory results are obtained e.g. for topical use with local administration of active substance at a concentration of from about 0.05 % to about 5 % by weight, preferably of from about 1 % to about 5 % by weight, especially of from about 1 % to about 3 % by weight several times daily, e.g. 2 to 5 times daily.

Examples of pharmaceutical compositions indicated for topical use are lotions, gels, ointments, paste and cremes, particularly gels and cremes.

**GALENICAL ASPECTS:**

The invention thus also includes pharmaceutical compositions suitable for anti-inflammatory, preferably topical use, comprising the compound of the invention in association with at least one pharmaceutical acceptable carrier or diluent.

It also includes pharmaceutical compositions comprising the compound of the invention in association with at least one pharmaceutically acceptable carrier or diluent; for use in the treatment, preferably topical, of inflammation.

Such compositions may be manufactured by mixing with a pharmaceutically acceptable carrier or diluent. Unit dosage forms contain, for example, from about 0.0025 mg to about 50 mg of active substance. The invention therefore also includes a process for the preparation of a pharmaceutical composition suitable for anti-inflammatory use comprising mixing the compound of the invention with a pharmaceutically acceptable carrier or diluent.

Some pharmaceutical compositions may be manufactured in conventional manner, e.g., for topical applications,

a) as a two-phase suspension system wherein the compound of the invention is present in at least partly undissolved state in the aqueous phase, e.g. as a gel as in Example 1 below;

b) as a two-phase suspension system wherein the compound is present on an exclusively organic supporting medium in at least partly undissolved state, e.g. as an ointment as in Example 2 below; or

c) as a system wherein the compound is dissolved in an organic supporting medium, e.g. as an ointment as in Examples 3 and 4 below.

The compound of the invention, however, exhibits unusual physicochemical properties, especially low solubility in water and oils; this makes difficult the preparation of galenical forms in general, and of galenical forms in particular in which the compound is dissolved in the supporting medium.

Thus the preparation of the topical galenical forms of choice for structurally related allylamine compounds such as naftifine (Exoderil$^R$) and terbinafine (Lamisil$^R$), namely of two-phase emulsion systems wherein the compound is present in dissolved state in the organic phase, including lotions, gels and cremes, is not applicable to the compound of the present invention. These topical galenical forms are prepared starting from an acid addition salt of the allylamine suspended in the watery phase of a two-phase system, and through the addition of an equimolar amount of a base such as sodium hydroxide the compound is driven into solution in free base form in the oily phase. With the compound of the invention, while the free base form is cristalline, it is not sufficiently soluble in either of the two phases of the two-phase system and thus crystallizes out upon cooling.

Pharmaceutical compositions prepared in conventional manner therefore may possess generally undesirable properties such as low resorption and uncertain bioavailability; further, where the compound is present in undissolved state, it has been observed that it forms particularly large, needle-shaped crystals of about 100 µm to about 300 µm in average length (see Figure 7). Thus topical applications are likely to be accompanied by discomfort, including possible skin irritation.

It has now been found that, surprisingly, these difficulties can be overcome, e.g. in the form of a pharmaceutical composition wherein the compound of the invention is present in dissolved state in an aqueous phase, e.g. of a one-phase hydrogel system as e.g. in Example 9 below or of a two-phase emulsion system as e.g. in Examples 5 to 9 below.

Among the pharmaceutical compositions which are part of this invention, the invention therefore includes especially pharmaceutical compositions wherein the compound of the invention is present in dissolved state in an aqueous phase, together with at least one pharmaceutically acceptable carrier or diluent, in particular one-phase hydrogel and two-phase emulsion systems wherein the compound is in the aqueous phase, e.g. lotions, gels and cremes.

Such compositions, as e.g. in Examples 5 to 9 below, are particularly indicated for topical use.

In the pharmaceutical compositions defined above the compound of the invention is dissolved in the aqueous phase in water-soluble acid addition salt form (see below), preferably as the lactate or ascorbate, especially the lactate.

Corresponding pharmaceutical compositions wherein the compound of the invention is present in dissolved state in an aqueous phase preferably include for topical use from about 1 % to about 5 % of the compound by weight, computed on the basis of the free base form, and an amount of water-soluble salt-forming reagent which is from about one-half to about four-fifths of that amount by weight; they include e.g. about 1 % by weight of compound of the invention together with a mixture of about 0.5 by weight of lactic acid and about 0.1 % by weight of ascorbic acid, or together with about 0.5 % by weight of lactic or ascorbic acid.

Pharmaceutical compositions incorporating the compound of the invention in water-soluble acid addition salt form may be manufactured by mixing the compound of the invention in free base form with appropriate pharmaceutically acceptable carriers or diluents under conditions such that a composition is formed wherein the compound is present in dissolved state in an aqueous phase, e.g. a one-phase hydrogel, or a two-phase emulsion system wherein the compound is in the aqueous phase. The invention therefore also includes a process for the preparation of a pharmaceutical composition comprising mixing the compound of the invention with pharmaceutically acceptable carriers or diluents under conditions such that a composition is formed wherein the compound is present in dissolved state in an aqueous phase, e.g. a one-phase hydrogel or a two-phase emulsion system wherein the compound is in the aqueous phase. The compound preferably is in the form of a water-soluble acid addition salt with a hydroxycarboxylic acid.

The above process may be effected in conventional manner, e.g. as follows:

The compound of the invention in free base form is preferably micronised, e.g. as described in Example 1 below. The salt-forming reagent and water are added and the mixture is stirred until a clear solution is formed. Then an antibacterial agent such as benzyl alcohol and more water are added to get the aqueous phase to its final volume. The oil phase is prepared separately by melting together appropriate consistency factors such as cetyl palmitate, cetyl alcohol or stearyl alcohol with isopropyl myristate and emulsifiers such as Arlacel 60$^R$ and Tween 60$^R$. The oil phase is then added to the aqueous phase, preferably at a temperature of about 70 $^\circ$C. Homogenization is followed by cooling down with continous stirring.

The compound of the invention being a phenol it can be expected to be susceptible to oxidation. Such oxidation can be inhibited by the use of anti-oxidants. A further embodiment of the above inventive concept thus includes, for water-soluble acid addition salt formation, using a salt-forming reagent which simultaneously can also act as an anti-oxidant, such as ascorbic acid as in Example 7 and 8 or, alternatively, adding an anti-oxidant such as sodium sulfite.

## WATER-SOLUBLE ACID ADDITION SALTS

Water-soluble forms of the compound of the invention were hitherto totally unknown. Thus the free base form of the compound of the invention and the naphthalene-1,5-disulfonate acid addition salt disclosed e.g. in USP 4'282'251 both have a solubility in water of less than 0.1 mg/ml at 25 $^\circ$C.

Water-soluble acid addition salt forms of the compound of the invention have now been found which are indicated for use e.g. in the above one-phase hydrogel or two-phase emulsion systems. Water-soluble acid addition salt forms are herein defined as acid addition salt forms of the compound of formula I as depicted above having a solubility in water of at least 1 mg/ml at 25 $^\circ$C. Preferably the solubility in water is of at least 14 mg/ml at 25 $^\circ$C, especially of at least 70 mg/ml at 25 $^\circ$C.

Examples of water-soluble acid addition salts are salts with hydroxycarboxylic acids. Preferred hydroxycarboxylic acids are $\alpha$-hydroxycarboxylic acids, preferably $\alpha$-hydroxycarboxylic acids which are used in dermatology as therapeutic adjuvants, e.g. lactic, ascorbic, tartaric, citric and malic acid, preferably lactic and ascorbic, especially lactic acid. Preferably the racemic form is used.

The preferred optically active form of lactic acid is the D-form. The preferred optically active form of ascorbic acid is the L-form. The preferred optically active form of tartaric acid is the D-form.

Although some $\alpha$-hydroxycarboxylic acids have been used in dermatology as therapeutic adjuvants, e.g. lactic acid, which is known to exert a moistening effect on the skin, they are rarely used in galenical applications. In particular, it is totally unexpected that these acids can form water-soluble salts with the compound of the invention. Thus the preparation of galenical forms wherein the compound of the invention is present in dissolved state in an aqueous phase, such as one-phase hydrogel or two-phase emulsion

systems wherein the compound is present in dissolved state in the aqueous phase, has now become possible.

The invention therefore also comprises the compound of formula I as depicted above in the form of a water-soluble acid addition salt as defined above.

The invention further also comprises a process for the preparation of the compound of formula I as depicted above in water-soluble acid addition salt form as defined above by reacting the compound of formula I in free base form with an appropriate acid addition salt-forming reagent and recovering the resultant salt.

The above process can be effected in conventional manner. The salt-forming reagent is any appropriate acid or acid derivative such as an anhydride or acyl halide. It is e.g. lactic acid or ascorbic acid when it is desired to form the lactate or, respectively, the ascorbate. The reagent is used in stoechiometric or, preferably, more than stoechiometric amount, e.g. from about 1 equivalent to about three equivalents of reagent, preferably from about 1.1 equivalent to about 2.0 equivalents, especially from about 1.5 to about 1.7 equivalents, e.g. 1.7 equivalents. The reaction normally is effected in water or a mixture of water and an alcohol such as ethanol or benzyl or cetyl alcohol. However, for galenical applications salt formation preferably is effected in situ, i.e. upon preparation of the galenical form, e.g. a lotion, gel or creme, which it is intended to use. Thus the starting material for galenical formulation generally is the compound of the invention in free base form. This form is well-characterized and can be easily cristallized as mentioned above; it can thus be used as a convenient starting material in a high state of purity.

When more than one salt-forming reagent is used, several water-soluble salt forms can be obtained simultaneously in varying proportions, e.g. a mixture of the lactate and the ascorbate, as in Examples 7 and 8 below.

The beneficial effects obtained with the above water-soluble acid addition salts, e.g. in pharmaceutical compositions wherein the compound of the invention is present in dissolved state in an aqueous phase, include more precise dosaging and the avoidance of skin irritation resulting from the presence of undissolved compound.

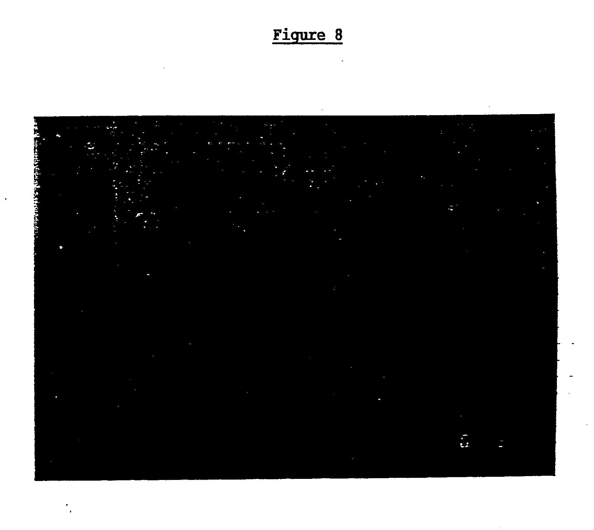

Thus the compound of the invention in free base form used i.a. in the preparation of the cream described in Example 5 below has the appearance visible in Figure 5 below before micronisation. When this material is recrystallized from ethanol/water it assumes the needle-shaped aspect visible in Figure 6 below. When the procedure of Example 5 is used without lactic acid, i.e. with b) omitted, in the cream obtained the compound is also recrystallized and assumes the same needle-shaped aspect as in Figure 6. This is evidenced in Figure 7 below. To the contrary, when the full procedure of Example 5 is followed, the compound is completely dissolved in the resultant cream, as is demonstrated in Figure 8 below.

## TOXICOLOGICAL ASPECTS

The compound of the invention has low toxicity; thus as regards acute toxicity in the mouse the $LD_{50}$ is expected to be above 1000 mg/kg p.o. and above 500 mg/kg i.p.

## E. EXAMPLES

The following Examples illustrate pharmaceutical compositions suitable for topical use and their preparation:

| **Example 1: Gel** | |
|---|---|
| Ingredient | Weight % |
| a) Compound of the invention (in free base form) | 1.0 |
| b) Hydroxypropylmethylcellulose (Methocel E4M) | 3.0 |
| c) Glycerol | 5.0 |
| d) Methylparaben | 0.07 |
| e) Propylparaben | 0.03 |
| f) $H_2O$ (pharmaceutical grade) | to 100.0 % |

a) is micronised employing an air-jet mill. Micronised product having a maximum particle size of 60-70 μm and an average particle size of from 2 to 10 μm is combined and mixed in conventional manner with the ingredients shown in the table above to provide an aqueous gel comprising 1 % by weight compound of the invention and suitable for topical application.

| **Example 2: Ointment** | | |
|---|---|---|
| Ingredient | Weight % | |
| | Composition A | Composition B |
| a) Compound of the invention (in free base form) | 1.0 | 5.0 |
| b) Anionic lanolin derivative (Amerchol CAB) | 2.0 | 2.0 |
| c) Liquid paraffin | 42.0 | 37.5 |
| d) White petrolatum | 55.0 | 55.5 |
| | 100.0 % | 100.0 % |

a) is prepared as for Example 1 above. b), c) and d) are melted together and stirred and a) is added at about 30° C, the particles being distributed employing an homogenizer. The obtained ointment is cooled and filled into collapsible tubes for topical application.

| **Example 3: Ointment** | |
|---|---|
| Ingredient | Weight % |
| a) Compound of the invention (in free base form) | 1.0 |
| b) Glycerol monostearate | 7.0 |
| c) Diethyleneglycol monoethyl ether (Transcutol$^R$) | 15.0 |
| d) Paraffin gelified with polyethylene (Plastibase$^R$) | 77.0 |
| | 100.0 % |

a) is prepared as for Example 1 above and is dissolved in c) with stirring and slightly warming up. c) and d) are melted together at about 60° C. The drug solution is then added with stirring and homogenizing and the product is cooled down to room temperature by continuous stirring to get an ointment.

| **Example 4: Ointment** | |
|---|---|
| Ingredient | Weight % |
| a) Compound of the invention (in free base form) | 1.0 |
| b) Glycerol monostearate | 7.0 |
| c) Polyethylene glycol | 15.0 |
| d) Paraffin gelified with polyethylene (Plastibase$^R$) | 77.0 |
| | 100.0 % |

The procedure of Example 3 is followed, using polyethylene glycol in place of Transcutol$^R$.

| Example 5: Cream | |
|---|---|
| Ingredient | Weight % |
| a) Compound of the invention (in free base form) | 1.0 |
| b) Lactic acid | 0.5 |
| c) Demineralised water | 71.5 |
| d) Antibacterial agent (benzyl alcohol) | 1.0 |
| e) Consistency factor (cetyl palmitate) | 2.0 |
| f) Consistency factor (cetyl alcohol) | 4.0 |
| g) Consistency factor (stearyl alcohol) | 4.0 |
| h) Isopropyl myristate | 8.0 |
| i) Emulsifier (Arlacel 60$^R$) | 1.9 |
| j) Emulsifier (Tween 60$^R$) | 6.1 |
| | 100.0 % |

a) is prepared as for Example 1 above. b) and about half the water c) are added and the mixture is stirred until a clear solution is obtained. Then the rest of water c) and d) are added to get the final water phase. This phase is warmed up to about 70 °C, e), f), g), h), i) and j) are molten together and warmed up to 70 °C to get the oil phase. This phase is then added to the hot water phase and emulsified using a homogenizer. Homogenizing is continued for 10 minutes and then the product is cooled down to room temperature with continuous stirring to get the final cream with the drug remaining dissolved in the water phase.

| Example 6: Cream | |
|---|---|
| Ingredient | Weight % |
| a) Compound of the invention (in free base form) | 5.0 |
| b) Lactic acid | 2.5 |
| c) Demineralised water | 65.5 |
| d) Antibacterial agent (benzyl alcohol) | 1.0 |
| e) Consistency factor (cetyl palmitate) | 2.0 |
| f) Consistency factor (cetyl alcohol) | 4.0 |
| g) Consistency factor (stearyl alcohol) | 4.0 |
| h) Isopropyl myristate | 8.0 |
| i) Emulsifier (Arlacel 60$^R$) | 1.9 |
| j) Emulsifier (Tween 60$^R$) | 6.1 |
| | 100.0 % |

The cream is prepared in a manner analogous to Example 5, using the amounts indicated above.

| Example 7: Cream | |
|---|---|
| Ingredient | Weight % |
| a) Compound of the invention (in free base form) | 1.0 |
| b) Lactic acid | 0.5 |
| c) Ascorbic acid | 0.1 |
| d) Demineralised water | 71.4 |
| e) Antibacterial agent (benzyl alcohol) | 1.0 |
| f) Consistency factor (cetyl palmitate) | 2.0 |
| g) Consistency factor (cetyl alcohol) | 4.0 |
| h) Consistency factor (stearyl alcohol) | 4.0 |
| i) Isopropyl myristate | 8.0 |
| j) Emulsifier (Arlacel $60^R$) | 1.9 |
| k) Emulsifier (Tween $60^R$) | 6.1 |
| | 100.0 % |

The cream is prepared in a manner analogous to Example 5, using the mixture of lactic and ascorbic acids in place of lactic acid alone.

| Example 8: Cream | |
|---|---|
| Ingredient | Weight % |
| a) Compound of the invention (in free base form) | 5.0 |
| b) Lactic acid | 0.5 |
| c) Ascorbic acid | 0.1 |
| d) Demineralised water | 65.4 |
| e) Antibacterial agent (benzyl alcohol) | 1.0 |
| f) Consistency factor (cetyl palmitate) | 2.0 |
| g) Consistency factor (cetyl alcohol) | 4.0 |
| h) Consistency factor (stearyl alcohol) | 4.0 |
| i) Isopropyl myristate | 8.0 |
| j) Emulsifier (Arlacel $60^R$) | 1.9 |
| k) Emulsifier (Tween $60^R$) | 6.1 |
| | 100.0 % |

The cream is prepared in a manner analogous to Example 5, using the mixture of lactic and ascorbic acids in place of lactic acid alone.

| Example 9: Gel | |
|---|---|
| Ingredient | Weight % |
| a) Compound of the invention (in free base form) | 1.0 |
| b) Lactic acid | 0.5 |
| c) Hydroxypropylmethylcellulose (Methocel E4M) | 3.0 |
| d) Glycerol | 5.0 |
| e) Methylparaben | 0.07 |
| f) Propylparaben | 0.03 |
| g) Water (pharmaceutical grade) | to 100.0 % |

Compound a) is suspended in part of water g). Lactic acid b) is added and the mixture stirred at room temperature until a clear solution is formed. e), f) and the rest of water g) are then added and the mixture is stirred with warming until all components are dissolved. In a separate vessel c) and d) are mixed together in

a way such that all particles are well wetted. Finally the aqueous solution of the other components is added and the mixture is stirred at room temperature until a clear hydrogel is formed.

The following Examples illustrate water-soluble acid addition salts and their preparation; such salts are indicated for use e.g. in pharmaceutical compositions wherein the compound of the invention is present in dissolved state in an aqueous phase:

**Example 10: Lactate**

50 mg of compound of the invention in free base form is suspended in 1 ml of distilled deuterated (heavy) water. 23 mg of a 90 % solution of lactic acid in heavy water are added under stirring at room temperature. After approximately 20 minutes a clear solution is formed. The presence of the lactate appears clearly from the NMR spectrum in deuterated dimethylsulfoxide (DMSO-$d_6$) (see Figure 1).

**Example 11: Lactate**

45 mg of compound of the invention in free base form is dissolved in 1 ml of deuterated dimethylsulfoxide (DMSO-$d_6$). 15 mg of a 90 % aqueous solution of lactic acid (equimolar amount) is added under stirring at room temperature. The NMR spectrum of this solution (see Figure 2), when compared with the NMR spectrum of the free base in DMSO-$d_6$ (see Figure 3) again demonstrates formation of the salt, e.g. from the chemical shift of the signals for the methyl and methylene groups adjacent to the nitrogen atom in the compound.

**Example 12: Lactate**

The procedure of Example 11 is repeated, using 50 mg compound of the invention in free base form and 23 mg (1.5 molar excess) of aqueous lactic acid solution. The corresponding NMR spectrum again shows formation of the salt (see Figure 4).

In all the Examples above "lactic acid" and "lactate" mean the racemic DL-form.

**Claims**

1. A water-soluble acid addition salt form of the compound of formula I

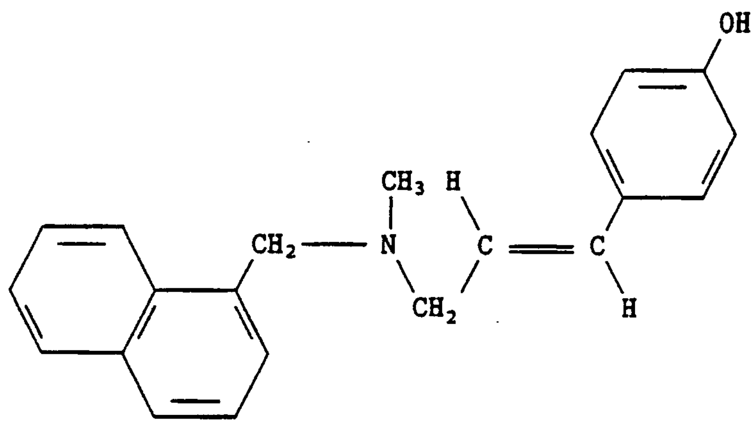

I

2. A compound salt form according to claim 1 which has a solubility in water of at least 14 mg/ml at 25 °C.

3. A compound salt form according to claim 1 which is a hydroxycarboxylic acid addition salt.

4. A compound salt form according to claim 1 which is the lactate.

5. A pharmaceutical composition suitable for anti-inflammatory use comprising the compound of formula I

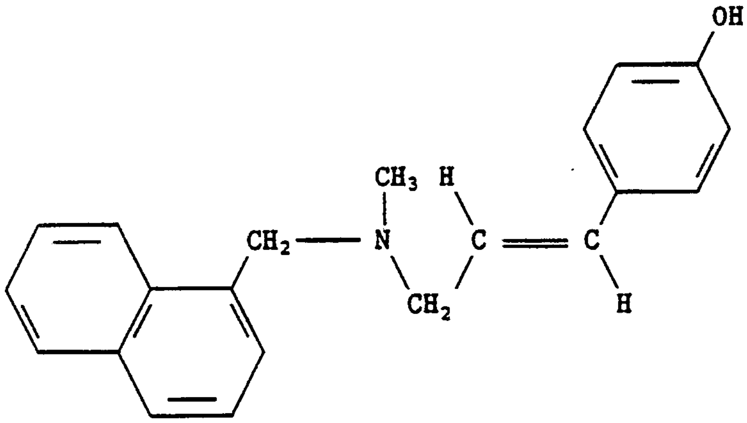

I

in free base form or in pharmaceutically acceptable acid addition salt form in association with at least one pharmaceutically acceptable carrier or diluent.

6. A pharmaceutical composition comprising a compound salt form according to claim 1 together with at least one pharmaceutically acceptable carrier or diluent.

7. A composition according to claim 6 wherein the compound salt form is present in dissolved state in an aqueous phase.

8. A process for the preparation of a compound salt form according to any one of claims 1 to 4 which comprises reacting the compound of formula I as depicted in claim 1 in free base form with an appropriate acid addition salt-forming reagent and recovering the resultant salt.

9. A process for the preparation of a pharmaceutical composition according to claim 5 comprising mixing the compound of formula I as depicted in claim 1 in free base form or in pharmaceutically acceptable acid addition salt form with a pharmaceutically acceptable carrier or diluent.

10. A process for the preparation of a pharmaceutical composition according to claim 7 comprising mixing the compound of formula I as depicted in claim 1 in free base form with appropriate pharmaceutically acceptable carriers or diluents under conditions such that a composition is formed wherein the compound is present in dissolved state in an aqueous phase.

Claims for the following Contracting States: ES, GR

1. A process for the preparation of a water-soluble acid addition salt form of the compound of formula I'

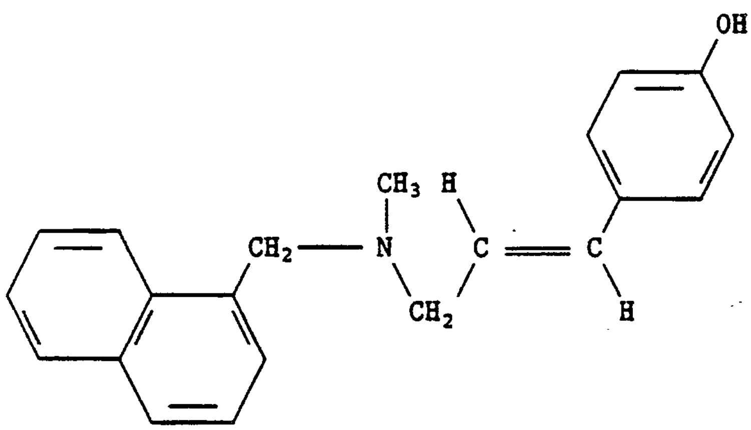

I

which comprises reacting the compound of formula I as depicted above in free base form with an appropriate acid addition salt-forming reagent and recovering the resultant salt.

2. A process for the preparation of a pharmaceutical composition suitable for anti-inflammatory use having the compound of formula I as depicted in claim 1 in free base form or in pharmaceutically acceptable acid addition salt form comprising mixing the compound of formula I as depicted in claim 1 in free base form or in pharmaceutically acceptable acid addition salt form with a pharmaceutically acceptable carrier or diluent.

3. A process for the preparation of a pharmaceutical composition having a compound salt form according to claim 1 comprising mixing the compound of formula I as depicted in claim 1 in free base form with appropriate pharmaceutically acceptable carriers or diluents under conditions such that a composition is formed wherein the compound is present in dissolved state in an aqueous phase.

Figure 1

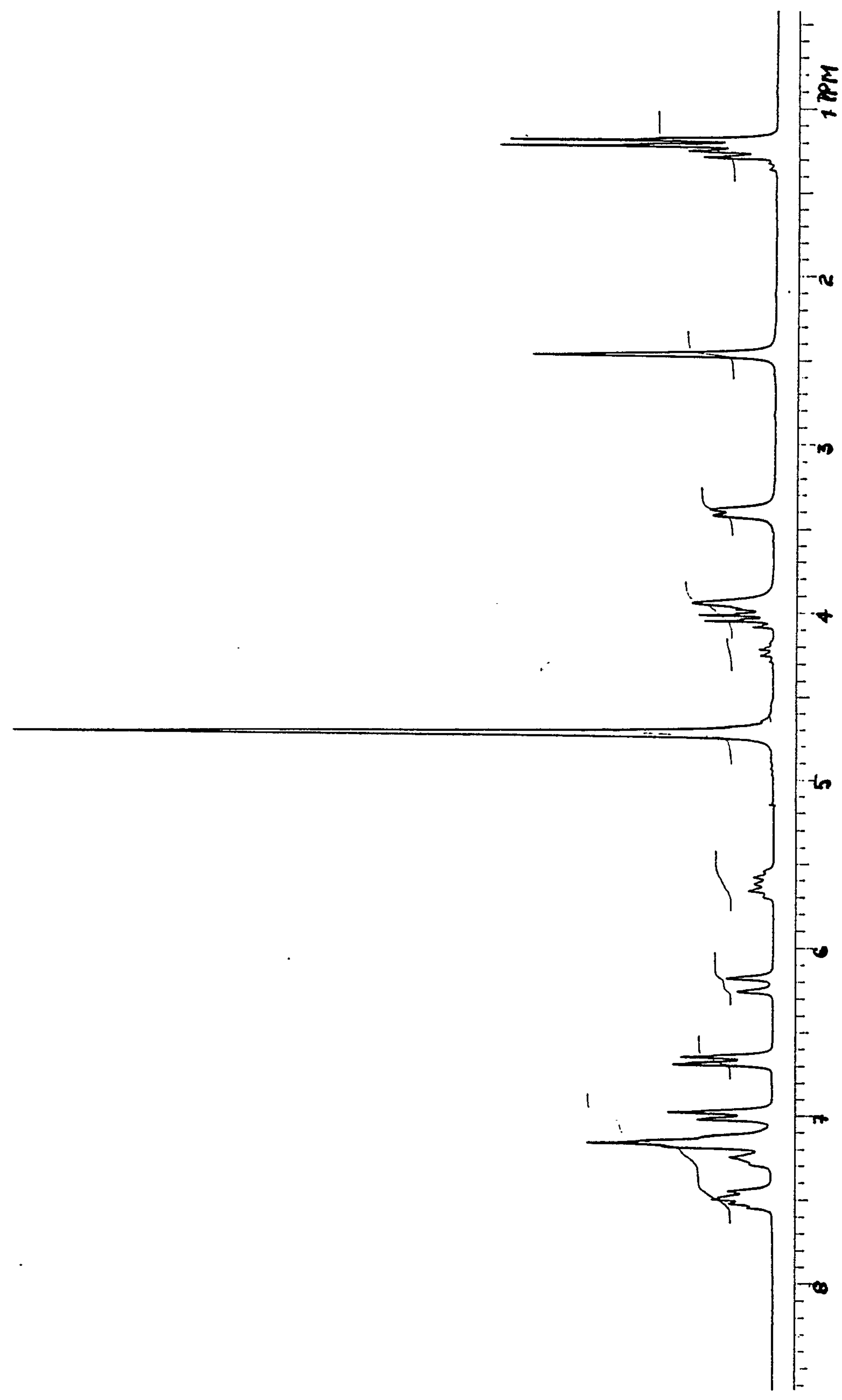

Figure 2

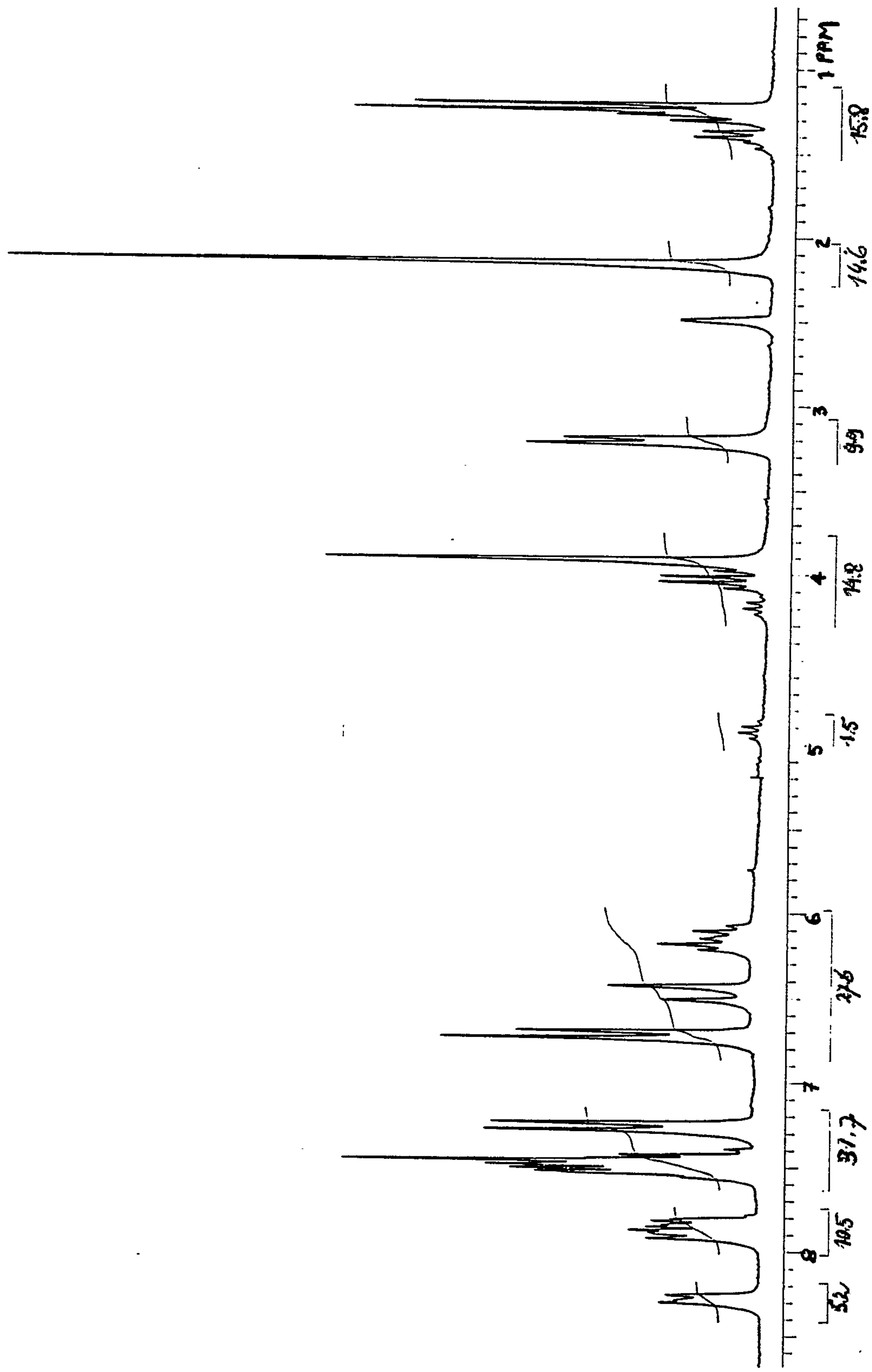

Figure 3

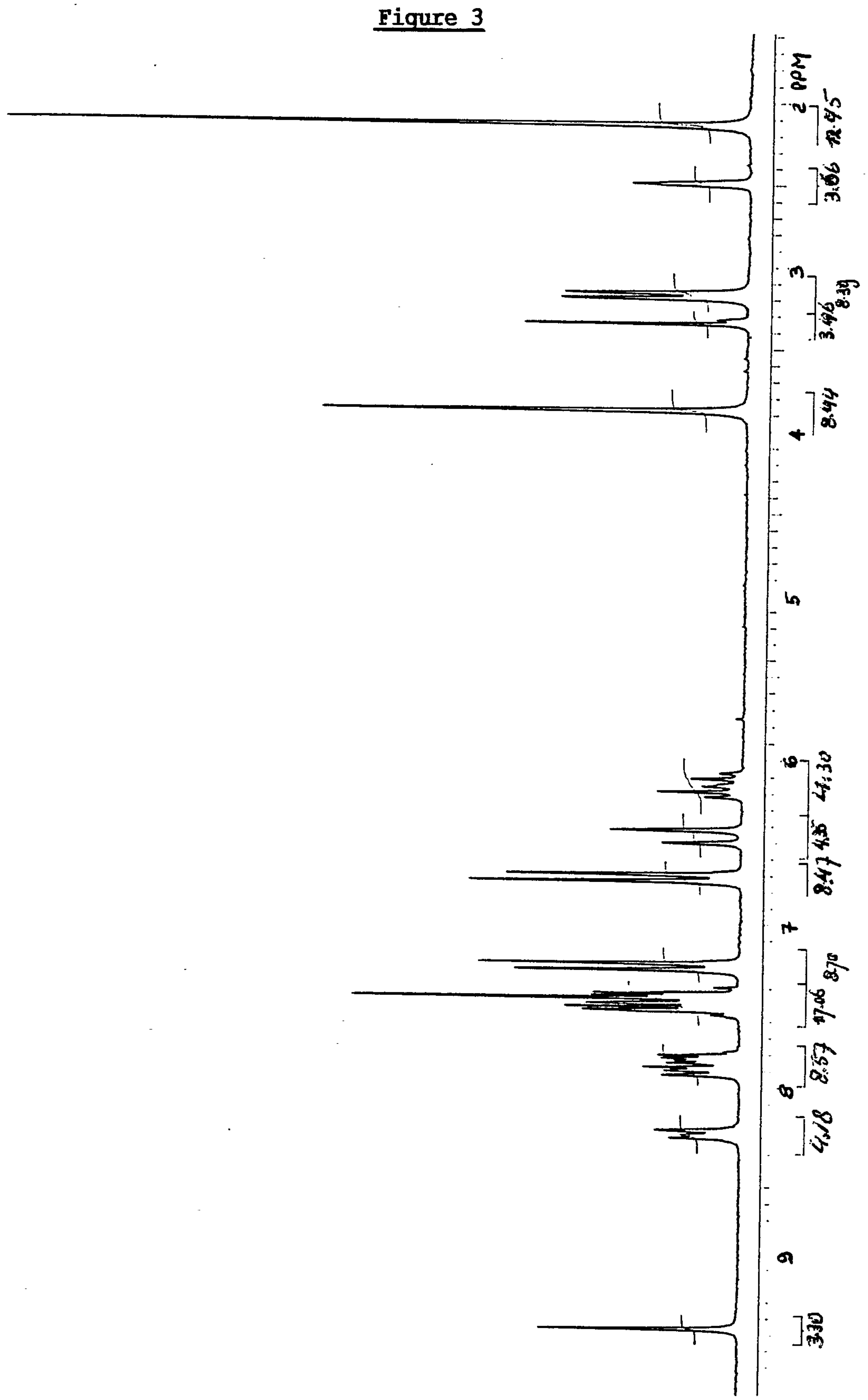

Figure 4

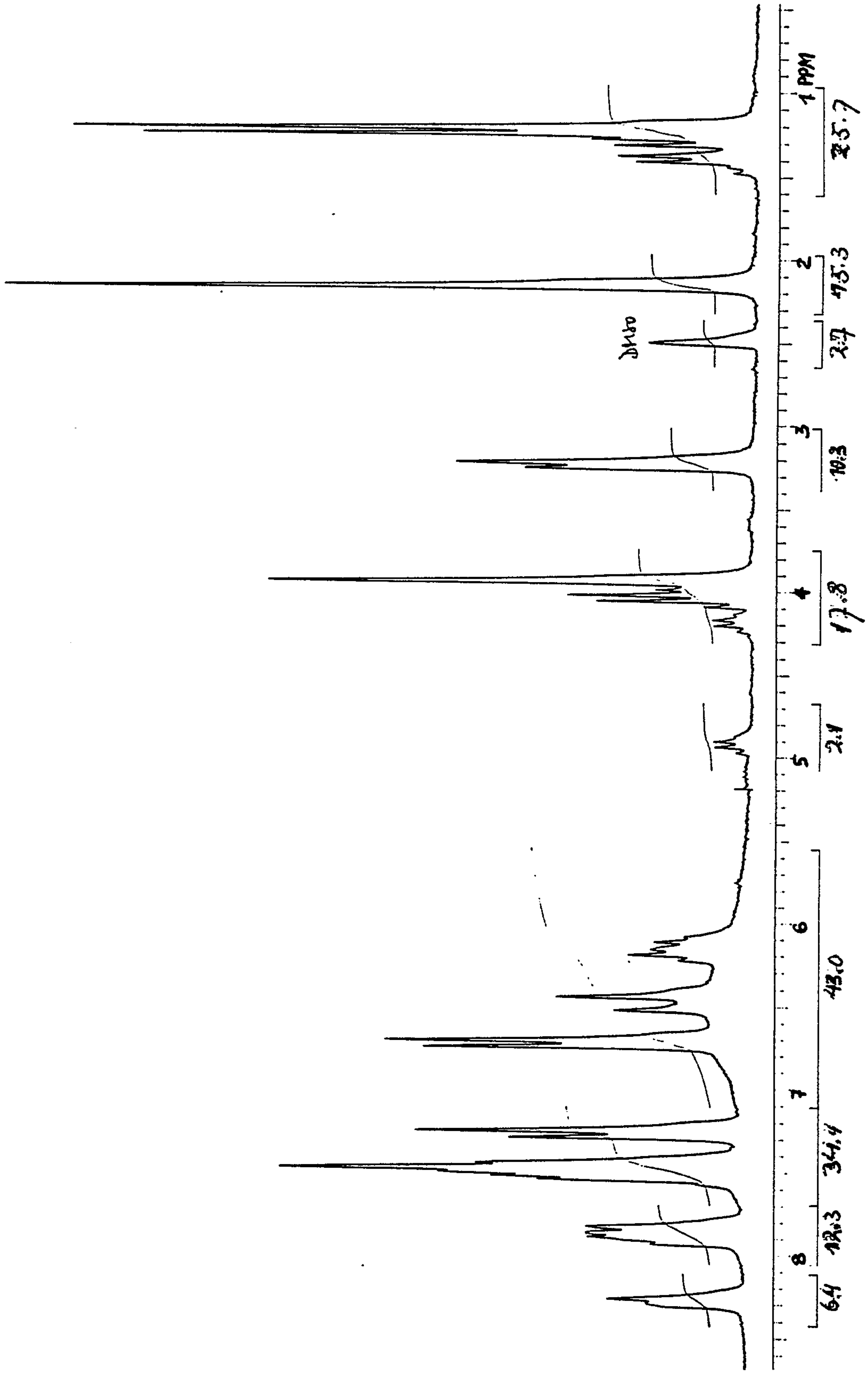

Figure 5

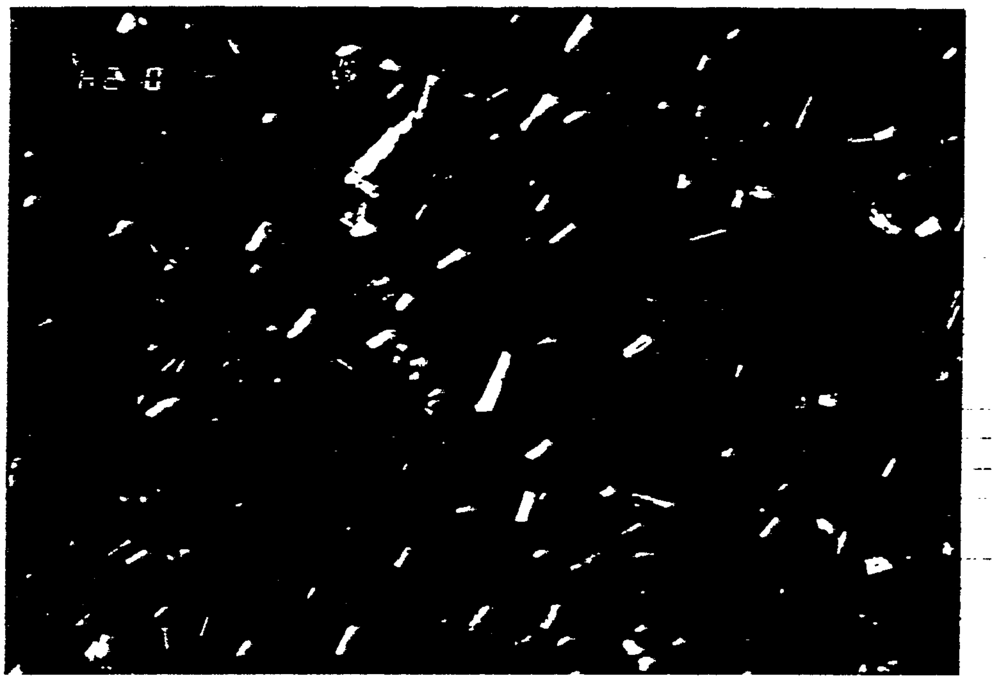

Figure 6

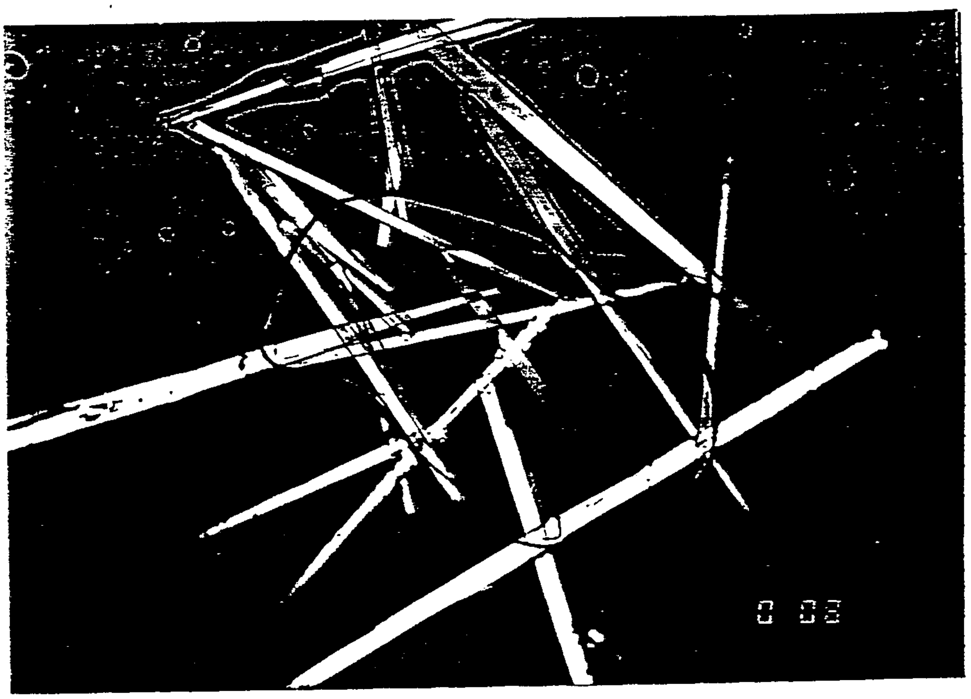

Figure 7

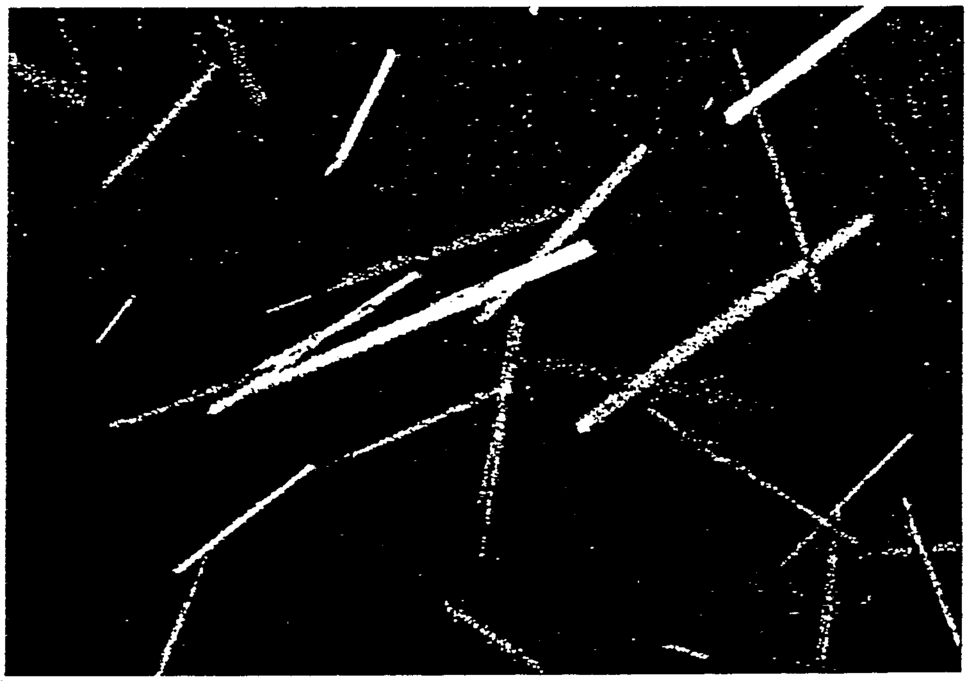

Figure 8